# EUROPEAN PATENT SPECIFICATION

(11) **EP 2 493 343 B1**
(45) Date of publication and mention of the grant of the patent: **06.11.2013**
(21) Application number: 10778724.4
(22) Date of filing: 28.09.2010
(51) Int. Cl.: A41D 13/12, A61M 16/08

(54) **GARMENT FOR TUBING MANAGEMENT**
KLEIDUNGSSTÜCK FÜR SCHLAUCHVERWALTUNG
VÊTEMENT POUR LA GESTION DE TUBES

(30) Priority: 28.10.2009 US 255585 P
(43) Date of publication of application: 05.09.2012
(73) Proprietor: Koninklijke Philips N.V., 5656 AE Eindhoven (NL)
(72) Inventor: MATULA JR., Jerome, Briarcliff Manor New York 10510-8001 (US); ANDREWS, Derrick Blake, Briarcliff Manor New York 10510-8001 (US); STARTARE, Anthony Vincent, Briarcilff Manor New York 10510-8001 (US)
(74) Representative: van Velzen, Maaike Mathilde
(86) International application number: PCT/IB2010/054370
(87) International publication number: WO 2011/051838

(56) References cited:
- WO-A1-2009/046523
- DE-U1-202007 003 133
- US-A1- 2008 210 236
- US-B1- 7 296 652

## Description

The present invention pertains to a method and apparatus for providing a garment structured to manage one or more tubes, and, in particular, a garment wherein the tube(s) are restricted to a longitudinal path along the garment.

People with breathing disorders, or other medical conditions, are often required to wear a patient interface device, such as a facemask, which is typically structured to supply air or oxygen to the airway of the user. The patient interface device is coupled to a gas supply conduit, which is further coupled to a gas source. The conduit, often identified as "tubing" or "patient circuit", typically extends from the gas source to patient interface device. As a result, if, for example, the tubing is located to the user's right and the user turns his/her head to the left, and if there is insufficient slack in the tubing, the tubing will not move with the user's head and the facemask may be pulled out of position. This often happens when the user is asleep as such system are often worn during sleep for the treatment of sleep apnea.

US 2008/0210236 discloses a tubing management system designed to be attached to a patient's shirt to reduce tube drag.

The present invention provides for a garment having a tubing management system incorporated therein. The tubing management system includes at least one conduit and a coupling system for the conduit. The coupling system has at least one coupling point, which is located at the neck opening of the garment. In an exemplary embodiment, the coupling system defines a path for the conduit, i.e., there is more than one attachment point, therefore the path of the conduit may be controlled. In this configuration, the conduit moves with the user and, as such, there is a reduced chance that the user's movements, even while asleep, will cause the facemask to be pulled from its proper position.

The coupling system is incorporated into the garment, but may also be simply coupled to the garment. The garment, which may be a shirt, a gown, pajamas, a robe, etc. is identified as a "covering." The coupling system on the covering is a loop through which the tubing management system conduit extends. This loop defines the path of the conduit. Moreover, as the loop is part of the covering, and, as the covering moves with the user, the conduit also moves with the user. The coupling system is in the form of an elongated loop that extends along the path of the conduit. For example, the elongated loop may be formed from a flap extending from the covering. The conduit is disposed between the flap and the covering, and the distal end of the flap is coupled to the covering. This defines a pocket through which the conduit extends. Alternately, the covering may have a first magnetic material incorporated therein. The conduit may then have a second magnetic material incorporated therein. When the magnetic materials are placed near each other, the conduit is magnetically coupled to the covering along a path defined by the first magnetic material.

There are a number of variations to these embodiments which are encompassed by the claimed concept. For example, rather than a single conduit, which may have a relatively large diameter, the conduit may be split into a number of smaller conduits. As each conduit in a plurality is thinner than a single conduit, the garment may allow for the user to, for example, roll over in their sleep without feeling the conduits. Further, the covering may incorporate padding over the conduit/conduits to further reduce the impact of the conduit on the user.

These and other objects, features, and characteristics of the present invention, as well as the methods of operation and functions of the related elements of structure and the combination of parts and economies of manufacture, will become more apparent upon consideration of the following description and the appended claims with reference to the accompanying drawings, all of which form a part of this specification, wherein like reference numerals designate corresponding parts in the various figures.

FIG. 1 is a schematic front view of a user wearing a garment with a non-claimed tubing management system;

FIG. 2 is a schematic front view of a user wearing an alternate embodiment of the garment with a tubing management system;

FIG. 3 is a schematic front view of a user wearing another alternate embodiment of the garment with a tubing management system;

FIG. 4 is a schematic front view of a user wearing another alternate embodiment of the garment with a tubing management system;

FIG. 5 is a schematic front view of a user wearing another alternate embodiment of the garment with a tubing management system;

FIG. 6 is a schematic front view of a user wearing another alternate embodiment of the garment with a tubing management system;

FIG. 7 is a schematic front view of a user wearing another alternate embodiment of the garment with a tubing management system;

FIG. 8 is a schematic front view of a user wearing another alternate embodiment of the garment with a tubing management system;

FIG. 9 is a schematic front view of a user wearing another alternate embodiment of the garment with a tubing management system; and

FIG. 10 is a schematic front view of a user wearing another alternate embodiment of the garment with a tubing management system.

As used herein, the singular form of "a", "an", and "the" include plural references unless the context clearly dictates otherwise. As used herein, "coupled" means a link between two or more elements, whether direct or indirect, so long as a link occurs. As used herein, "directly coupled" means that two elements are directly in contact with each other. As used herein, "fixedly coupled" or "fixed" means that two components are coupled so as to move as one while maintaining a constant orientation relative to each other. As used herein, the word "unitary" means a component is created as a single piece or unit. That is, a component that includes pieces that are created separately and then coupled together as a unit is not a "unitary" component or body. As used herein, a "facemask" includes any type of oral, oral-nasal, or nasal mask or nose-plug, structured to provide a gas to a user. As used herein, a "matrix" includes random as well as symmetric/regular constructs.

As used herein, a "first magnetic material" and a "second magnetic material" include a material that is attracted to iron or steel, e.g., a typical magnet, and material such as iron, steel, or another magnet to which the first material is attracted. It is understood that the purpose of the "first magnetic material" and the "second magnetic material" is to have the materials magnetically attracted to each other. Thus, two non-magnetized materials, e.g. two pieces of normal steel, are not a "first magnetic material" and a "second magnetic material."

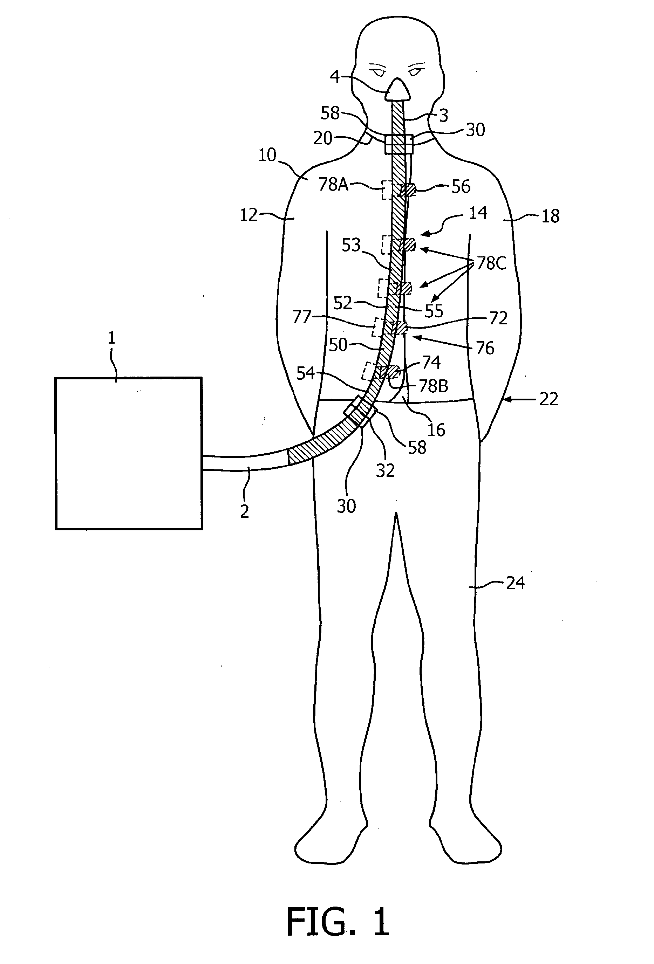

A garment 10 structured to cooperate with a gas supply system 1 is shown in FIG. 1. As is known, the gas supply system 1 includes a supply conduit 2 and a facemask conduit 3 as well as a facemask 4. Supply conduit 2 is structured to be coupled to, and in fluid communication with, a gas supply system 1. Facemask conduit 3 is structured to be coupled to, and in fluid communication with facemask 4. Typically, the gas exhaled by the user is exhausted to the atmosphere. If, however, the gas exhaled by the user is to be collected, or otherwise routed away from the user, facemask 4, supply conduit 2, facemask conduit 3, facemask 4, and the tubing management system at least one conduit 50 (discussed below) may each include one or more exhaust vents (not shown).

Garment 10 includes a covering 12 and a tubing management system 14. Covering 12 is, generally, shown in the FIGS. as a shirt. Covering 12 may, however, be any type of covering such as, but not limited to, a gown, pajamas, a robe, tank-top, etc., that is, generally any type of clothing in which a person typically sleeps. Covering 12 is made from a thin, flexible material, such as cloth. Covering 12 has an inner side 16 and an outer side 18. Covering 12 is structured to be disposed over at least the upper part of a body. Covering 12 further has a neck opening 20 and a waist portion 22. As shown in FIGS. 1-2, covering 12 may include leggings 24, which may be separate from, or unitary with, the upper portion of the covering 12.

Tubing management system 14 has a coupling system 30 and at least one conduit 50. Coupling system 30 is structured to couple the tubing management system at least one conduit 50 to covering 12 at least one coupling point 32. The at least one coupling point 32 is at covering neck opening 20 at the front of the neck opening. The tubing management system at least one conduit 50 is structured to extend from at least covering waist portion 22 to covering neck opening 20. The tubing management system also includes at least one conduit 50 is structured to be in fluid communication with both supply conduit 2 and facemask conduit 3. Coupling system 30 is adapted to operate with the type of tubing management system at least one conduit 50 that is used.

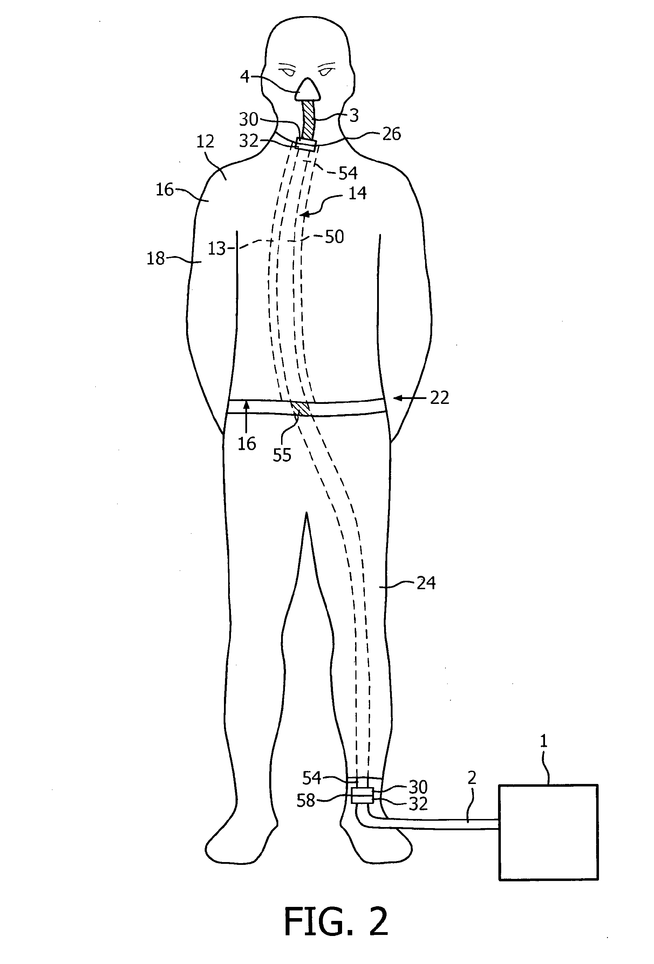

As shown in FIG. 2, the tubing management system at least one conduit 50 is at least one flexible tube 52. The at least one flexible tube 52 may be a single elongated, tubular member 53 having a lower, first end 54 and an upper, second end 56. The at least one flexible tube 52 has a conduit interface 58, i.e., a detachable coupling, at both first and second ends 54, 56. Each conduit interface 58 is structured to be removably coupled to the supply conduit 2 or the facemask conduit 3. In this manner, the at least one flexible tube 52 may be detached from either, or both, the supply conduit 2 or the facemask conduit 3. Thus, a user may, for example, detach the at least one flexible tube lower, first end 54 from the supply conduit 2 so that the user may travel to a restroom.

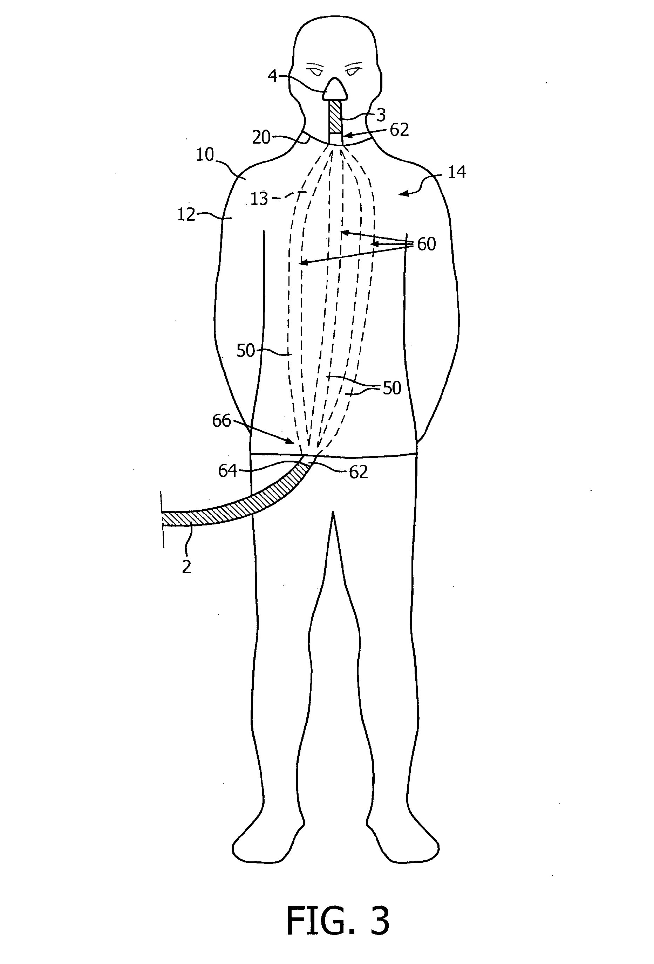

Alternatively, the tubing management system conduit at least one flexible tube 52 may be a plurality of smaller elongated tubes 60, as shown in FIG. 3. In this embodiment, each conduit interface 58 includes a manifold 62. That is, manifold 62 is a collar-like body having a larger, single opening 64 and a plurality of smaller openings 66. At the lower conduit interface 58, manifold larger opening 64 is coupled to, and in fluid plurality of smaller openings 66 is coupled to, and in fluid communication with, one of the plurality of smaller elongated tubes 60 and manifold larger opening 64 is coupled to, and in fluid communication with, facemask conduit 3.

For either of these two embodiments, i.e., the at least one flexible tube 52 being either a single tube 53 or a plurality of tubes 60, coupling system 30 may be at least one loop coupling 70 coupled to each at least one flexible tube 52, as shown in FIG. 1 but not forming part of the claimed invention. The non-claimed at least one loop coupling 70 forms a loop 72 about the at least one flexible tube 52. Loop 72 may be formed entirely separate from the covering 12, e.g. a metal ring (not shown) coupled to the covering 12 by a strap, or loop 72 may be formed by a strap 74 coupled at either end to the covering 12. That is, loop 72 is formed by both the covering 12 and the strap 74. Further, the at least one loop coupling 70 may be disposed on the covering inner side 16 or the outer side 18. It is noted that, when the at least one loop coupling 70 is disposed on the covering inner side 16, covering 12 may include padding 13 disposed between covering 12 and the user. Further, it is noted that the at least one loop coupling 70 may include a non claimed plurality of relatively thin loops 78 and a claimed elongated loop 80.

Thus, the non-claimed at least one loop coupling 70 may be at least two thin loop couplings 78, formed by a strap 74 and a coupling device 76, such as but not limited to, buckles 77, hook-and-loop fabric 94 (discussed below), snaps (not shown), or buttons. As an example, one loop coupling 78A is disposed at covering neck opening 20 and one loop coupling 78B is disposed at covering waist portion 22. However, there are a plurality of loop couplings 78C disposed between neck opening loop coupling 78A and waist portion loop coupling 78B.

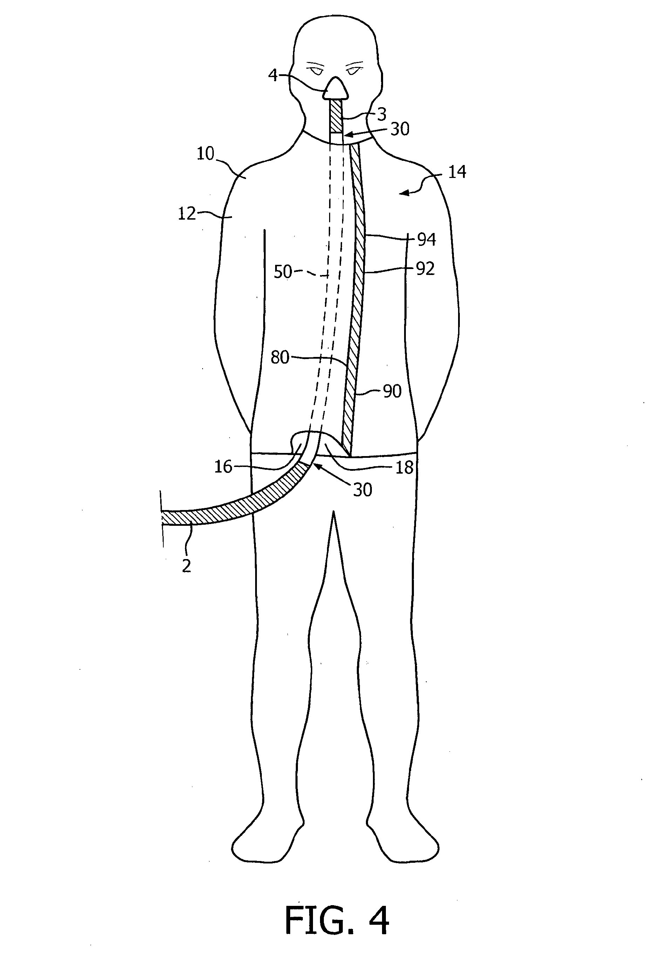

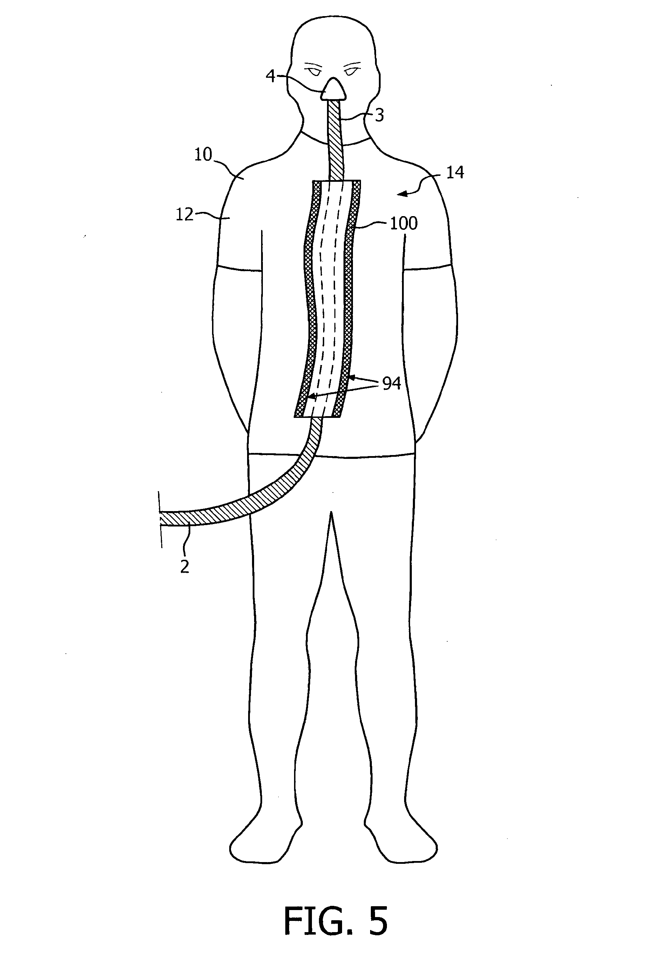

As shown in FIGS. 4 and 5, for either of these two embodiments, i.e., the at least one flexible tube 52 being either a single tube 53 or a plurality of tubes 60, coupling system 30 is an elongated loop 80 extending, substantially, between the neck opening 20 and the waist portion 22. That is, the at least one flexible tube 52 has a medial portion 55 disposed between the at least one flexible tube first and second ends 54, 56. The at least one flexible tube medial portion 55 may be substantially enclosed within an elongated loop 80, which may also be identified as a pocket, as discussed below. For example, as shown in FIG. 4, the covering 12 may include a flap 90 of material extending from the covering neck opening 20 to the covering waist portion 22. Flap 90 has a distal end 92 with a hook-and-loop fabric 94 coupled thereto. A strip of the corresponding hook-and-loop fabric 94 is coupled to the covering 12. Thus, the user may dispose the at least one flexible tube 52 between covering 12 and flap 90, and couple the flap to the covering using the hook-and-loop fabric 94. This forms a pocket-like elongated loop 80 through which the at least one flexible tube 52 extends. It is noted that, while FIG. 4 shows the at least one flexible tube 52 as being a single tube 53, flap 90 may also be used to enclose a plurality of tubes 60. If the at least one flexible tube 52 is a plurality of tubes 60, flap 90 may have an extended width, so that the plurality of tubes 60 may be disposed in a generally flat pattern.

Elongated loop 80 may also be created from a separate element rather than a flap 90, as shown in FIG. 5. That is, whereas a flap 90 has one edge that is unitary with the covering 12, the separate element may be an elongated strip of fabric 100 with two strips of hook-and-loop fabric 94 disposed on each elongated edge. Covering 12, has corresponding strips of hook-and-loop fabric 94 extending generally between the covering neck opening 20 to the covering waist portion 22. Thus, when the elongated strip of fabric 100 is coupled to covering 12 by the strips of hook-and-loop fabric 94, an elongated loop 80, or pocket, is formed. The at least one flexible tube 52 is disposed with the elongated loop 80.

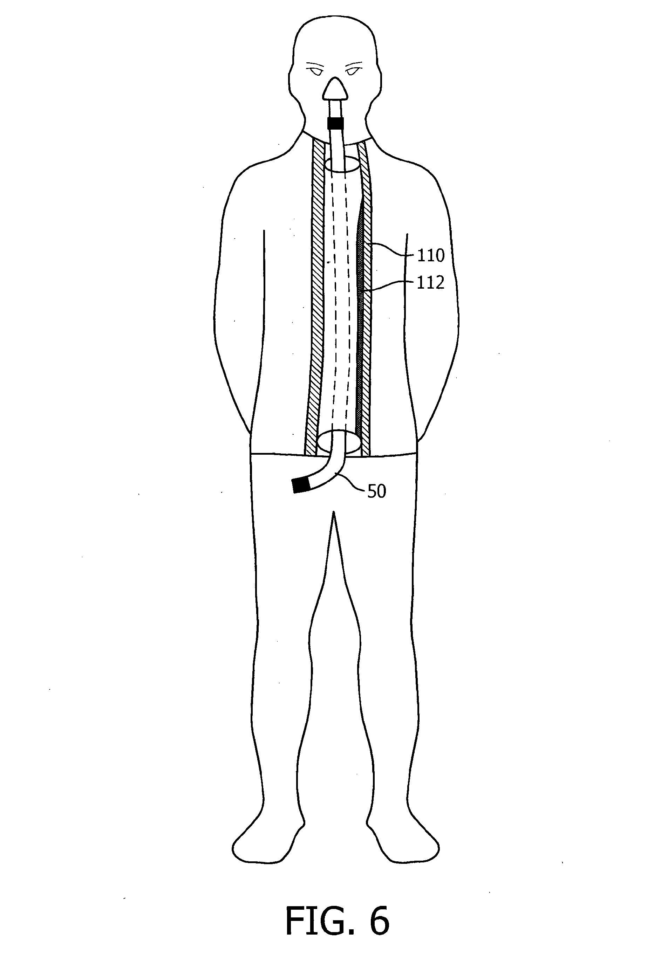

In a similar manner, elongated loop 80 may be formed of a separate element that is a magnetic material, as shown in FIG. 6. That is, covering 12 may have a first magnetic material 110 incorporated therein. First magnetic material 110 may be limited to a portion, e.g. the front, of covering 12. In this embodiment, rather than two strips of hook-and-loop fabric 94, elongated strip of fabric 100 may have two strips of a second magnetic material 112 disposed on each elongated edge. Thus, when elongated strip of fabric 100 is coupled to covering 12 by the strips of a second magnetic material 112, an elongated loop 80, or pocket, is formed. The at least one flexible tube 52 is disposed with elongated loop 80.

In either embodiment having an elongated strip of fabric 100, i.e., the embodiment with strips of hook-and-loop fabric 94 or the embodiment with strips of a second magnetic material 112, the at least one flexible tube 52 may also be adhered to, or otherwise coupled to, the strip of fabric 100 and may be disposed outside of the elongated loop 80. For example, rather than strip of fabric 100 having two strips of a second magnetic material 112 disposed on each elongated edge, second magnetic material 112 may be in the form of an elongated, flexible sheet 120 of second magnetic material 112. Sheet 120 of second magnetic material 112 may be, but is not limited to, a flexible rubber having the second magnetic material incorporated therein. The at least one flexible tube medial portion 55 may be fixed, or otherwise coupled, to the elongated, flexible sheet 120 of second magnetic material 112.

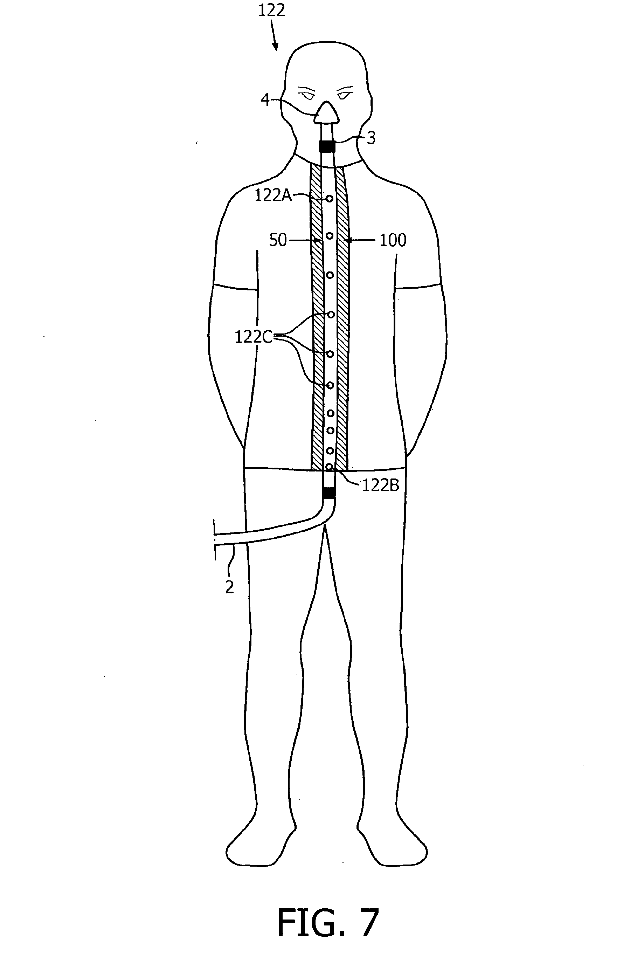

In another embodiment utilizing magnets, second magnetic material 112 may be incorporated into the at least one flexible tube 52, as shown in FIG. 7. As before, covering 12 may have a first magnetic material 110 incorporated therein. Frst magnetic material 110 may be limited to a portion, e.g. the front, of the covering 12. In this embodiment, second magnetic material 112 is in the form of at least one slug 122 fixed to the at least one flexible tube 52. As with loop couplings 78, discussed above, in an exemplary embodiment, one slug 122A is disposed at covering neck opening 20 and one slug 122B is disposed at covering waist portion 22. In a further embodiment, there are a plurality of slugs 122C disposed between neck opening slug 122A and waist portion slug 122B.

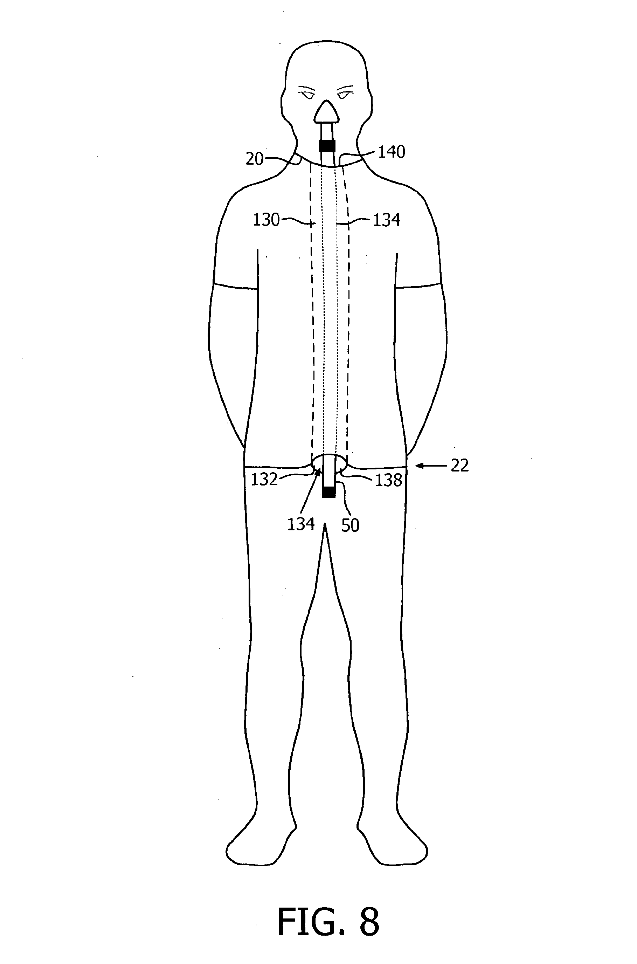

In another embodiment, shown in FIG. 8, which is similar to the embodiments having a flap 90 or elongated strip of fabric 100, i.e., the embodiment with strips of hook-and-loop fabric 94 or the embodiment with strips of a second magnetic material 112, a pocket may be incorporated into the covering 12 itself. That is, covering 12 may include at least a portion 130 with an inner layer 132 and an outer layer 134 of fabric. Inner and outer layers 132, 134 form at least one plenum 136 therebetween. The at least one plenum 136 has a first, lower opening 138 disposed at the waist portion 22, and, a second, upper opening 140 disposed at the neck opening 20. In this embodiment, the plenum 136 is the coupling system 30 with the at least one flexible tube 52 extending therethrough. As before, if the at least one flexible tube 52 includes a plurality of flexible tubes 60, plenum 136 may be made with a greater width similar to the width shown in FIG. 3.

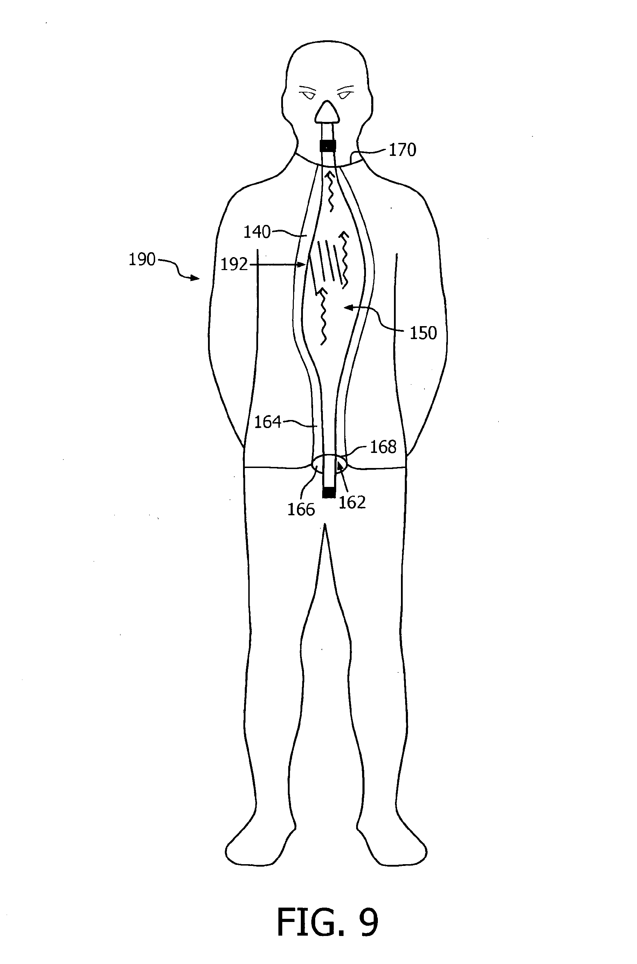

In another embodiment, shown in FIG. 9, the tubing management system at least one conduit 50 may be a bladder 150. Similar to the plenum 136 described above, covering 12 may include at least one portion 160 with an inner layer 162 and an outer layer 164 of fabric. The fabric of the bladder 150, however, is substantially impervious to gas. Further, bladder 150 includes a first, lower opening 166 having a conduit interface 168 structured to be coupled to the supply conduit 2, and second, upper opening 170 having a conduit interface 168 structured to be coupled to the facemask conduit 3. Thus, bladder 150 is structured to be coupled to, and in fluid communication with, both the supply conduit 2 and the facemask conduit 3. Accordingly, a gas may be transferred to/from the facemask 4 through the covering 12.

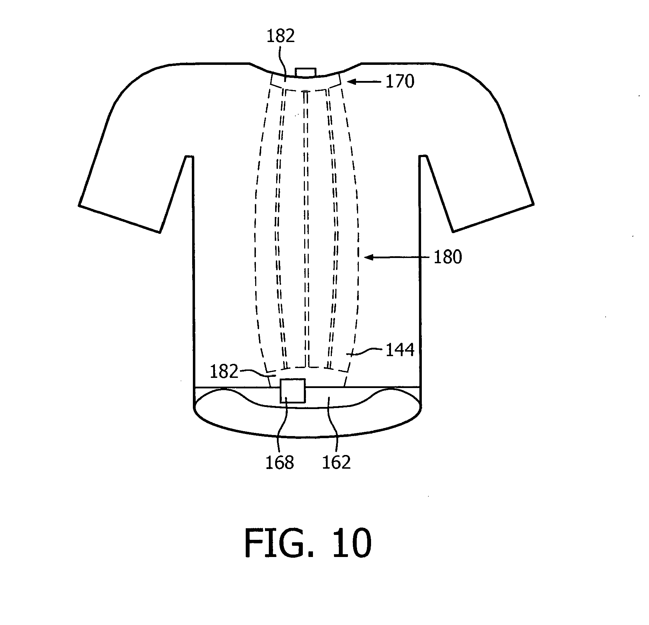

Just as the single elongated, tubular member 53 may be replaced by a plurality of smaller elongated tubes 60, single bladder 150 may be replaced either by a plurality of smaller bladders 180, as shown in FIG. 10. That is, the at least one portion 160 of the covering 12 with an inner layer 162 and an outer layer 164 of fabric may include a plurality of portions having the two layers 162, 164, each portion extending from the first, lower opening 166 to the second, upper opening 170. It is noted that bladders 180 may include manifold bladders 182 disposed about covering neck opening 20 and covering waist portion 22 which act as manifolds. Manifold bladders 182 are in fluid communication with each of the smaller bladders 180. Thus, if the user collapses one of the smaller bladders 180, gas may flow through manifold bladders 182 and pass through the open smaller bladders.

Further, any of the single bladder 150, smaller bladders 180 or manifold bladders 182 may include a matrix 190 of a rigid material structured to prevent total restriction of fluid flow through the bladder 150, 180, 182. Matrix 190 may be, but is not limited to, a plurality of ridges 192, or tubular members (not shown) extending through the bladder 150, 180, 182. Alternately, matrix 190 may be composed of a particulate material, e.g. a plurality of beads (not shown), so long as the particles have a sufficient size so as to not block fluid flow through the bladder 150, 180, 182.

It is noted that in each of the Figures, the path of the tubing management system at least one conduit 50 is shown as being centrally located at the front of the garment 10. The path of the tubing management system at least one conduit 50, however, may extend along various routes. For example, a person who tends to sleep on their stomach may prefer that the tubing management system at least one conduit 50 extend along the back of the garment 10. As such, the disclosed garment is not limited to an embodiment wherein the path of the tubing management system at least one conduit 50 extends over the front and center of the garment 10.

Although the invention has been described in detail for the purpose of illustration based on what is currently considered to be the most practical and preferred embodiments, it is to be understood that such detail is solely for that purpose and that the invention is not limited to the disclosed embodiments, but, on the contrary, is intended to cover modifications and equivalent arrangements that are within the scope of the appended claims. For example, it is to be understood that the present invention contemplates that, to the extent possible, one or more features of any embodiment can be combined with one or more features of any other embodiment.

## Claims

1. A garment (10) comprising:
(a) a covering (12) structured to be disposed over at least a portion of the upper body, wherein the covering includes an inner side (16), an outer side (18), a neck opening (20), and a waist portion (22); and
(b) a tubing management system (14) including:
(1) a conduit (50) extending from the waist portion to the neck opening (20), the conduit (50) comprising a flexible tube (52) coupled to the inner side or the outer side of the covering, wherein the conduit (50) is adapted to be detachably coupled to a supply conduit (2) and a facemask conduit (3); and
(2) a coupling system (30) adapted to couple the conduit to the covering,
**characterised in that** the coupling system (30) includes a first loop coupling (70) coupled to the flexible tube and the covering, the first loop coupling including an elongated loop (80) extending between the neck opening (20) and the waist portion (22).

2. The garment of claim 1, wherein the conduit (50) includes a lower first manifold (62), a plurality of flexible tubes (60), and an upper second manifold (62), wherein the upper second manifold (62) is structured to be coupled to the facemask conduit (3).

3. The garment of claim 1, wherein the conduit (50) includes a flexible tube (52) having has a lower first end, a medial portion, and an upper second end, and wherein the coupling system (30) includes a first magnetic material (110) operatively coupled to a portion of the covering (12) and a second magnetic material (112) operatively coupled to a portion of the conduit (50) such that the first magnetic material (110) and the second magnetic material (112) interact to couple the conduit (50) to the covering (12).

4. The garment of claim 3, wherein conduit (50) includes a at least one slug (122) made from the second magnetic material (112), wherein the at least one slug (122) is fixed to the flexible tube (52).

5. The garment of claim 1, wherein the coupling system (30) includes an elongated sheet of a first magnetic material (110) adapted to be coupled to the covering (12), wherein the conduit includes a lower first manifold (62), a plurality of medial flexible tubes (60), and an upper second manifold (62), wherein the upper second manifold (62) is structured to be coupled to such a facemask conduit (3), and wherein the coupling system (30) further includes a sheet of a second magnetic material (112) structured so as to be coupled to the sheet of a first magnetic material (110) with the medial tubes (60) disposed therebetween.

6. The garment of claim 1, wherein the covering (12) has at least a portion (16) with an inner layer (162) and an outer layer (164), the inner and the outer layers forming a plenum (136) therebetween, wherein the plenum (136) has a first lower opening (166) disposed proximate to the waist portion (22), and a second upper opening (170) disposed proximate to the neck opening (20), and wherein the plenum (136) is the coupling system (30).

7. The garment of claim 6, wherein the conduit (50) is at least one flexible tube (32) extending through the plenum (136).

8. The garment (10) of claim 6, wherein the conduit (50) includes a lower first manifold (62), a plurality of medial flexible tubes (60), and an upper second manifold (62), wherein the upper second manifold (62) is adapted to be coupled to such a facemask conduit (3), and wherein the plurality of medial flexible tubes (60) extend through the plenum (136).

9. The garment of claim 6, wherein the inner layer (162) and the outer layer (164) include a gas impervious material and the plenum forms a bladder (150) defining the conduit (50), and wherein the conduit includes:
a first lower opening (166) having a conduit interface (168) structured to be coupled to such a supply conduit (2), and
a second upper opening (170) having a conduit interface (168) structured to be coupled to such a facemask conduit (3).

10. The garment of claim 9, wherein the bladder (150) is filled with a matrix of rigid material.

11. The garment of claim 6, wherein the inner layer (162) and the outer layer (164) include a gas impervious material and the plenum (136) forms a plurality of bladders (180) defining the conduit (50).

12. The garment of claim 1, wherein the covering (12) further includes leggings (24) and the conduit (50) further extends through at least a portion of the leggings (24).

## Patentansprüche

1. Kleidungsstück (10), das Folgendes umfasst:
(a) eine Hülle (12), die so strukturiert ist, dass sie zumindest einen Teil des Oberkörpers umgibt, wobei die Hülle eine Innenseite (16), eine Außenseite (18), eine Halsöffnung (20) und einen Taillenteil (22) umfasst, und
(b) ein Schlauchverwaltungssystem (14), das Folgendes umfasst:
(1) eine Leitung (50), die sich vom Taillenteil bis zur Halsöffnung (20) erstreckt, wobei die Leitung (50) einen Schlauch (52) umfasst, der mit der Innenseite oder der Außenseite der Hülle verbunden ist, wobei die Leitung (50) für die lösbare Ankopplung an eine Zuführleitung (2) und eine Gesichtsmaskenleitung (3) ausgelegt ist, und
(2) ein Ankopplungssystem (30), das für die Ankopplung der Leitung an die Hülle ausgelegt ist, **dadurch gekennzeichnet, dass** das Ankopplungssystem (30) eine erste Schleifenkupplung (70) umfasst, die mit dem Schlauch und der Hülle verbunden ist, wobei die erste Schleifenkupplung eine längliche Schleife (80) umfasst, die sich von der Halsöffnung (20) bis zum Taillenteil (22) erstreckt.

2. Kleidungsstück nach Anspruch 1, wobei die Leitung (50) ein unteres erstes Anschlussstück (62), eine Vielzahl von Schläuchen (60) und ein oberes zweites Anschlussstück (62) umfasst, wobei das obere zweite Anschlussstück (62) so strukturiert ist, dass es mit der Gesichtsmaskenleitung (3) verbunden wird.

3. Kleidungsstück nach Anspruch 1, wobei die Leitung (50) einen Schlauch (52) mit einem unteren ersten Ende, einem Mittelteil und einem oberen zweiten Ende umfasst und wobei das Ankopplungssystem (30) ein erstes magnetisches Material (110), das operativ an einen Teil der Hülle (12) gekoppelt ist, und ein zweites magnetisches Material (112), das operativ an einen Teil der Leitung (50) gekoppelt ist, umfasst, sodass das erste magnetische Material (110) und das zweite magnetische Material (112) in Wechselwirkung stehen und die Leitung (50) an die Hülle (12) koppeln.

4. Kleidungsstück nach Anspruch 3, wobei die Leitung (50) zumindest ein Blöckchen (122) aus dem zweiten magnetischen Material (112) umfasst, wobei das zumindest eine Blöckchen (122) an dem Schlauch (52) befestigt ist.

5. Kleidungsstück nach Anspruch 1, wobei das Ankopplungssystem (30) eine längliche Platte aus einem ersten magnetischen Material (110) umfasst, die für die Kopplung an die Hülle (12) ausgelegt ist, wobei die Leitung ein unteres erstes Anschlussstück (62), eine Vielzahl in der Mitte befindlicher Schläuche (60) und ein oberes zweites Anschlussstück (62) umfasst, wobei das obere zweite Anschlussstück (62) so strukturiert ist, dass es an eine derartige Gesichtsmaskenleitung (3) gekoppelt wird, wobei das Ankopplungssystem (30) ferner eine Platte aus einem zweiten magnetischen Material (112) umfasst, die so strukturiert ist, dass sie an die Platte aus einem ersten magnetischen Material (110) gekoppelt wird, wobei die in der Mitte befindlichen Schläuche (60) dazwischen angeordnet sind.

6. Kleidungsstück nach Anspruch 1, wobei die Hülle (12) zumindest einen Teil (116) mit einer Innenschicht (162) und einer Außenschicht (164) umfasst, wobei die Innen- und die Außenschicht dazwischen einen ausgefüllten Raum (136) bilden, wobei der ausgefüllte Raum (136) eine erste untere Öffnung (166) aufweist, die nahe dem Taillenteil (122) angeordnet ist, und eine zweite obere Öffnung (170) aufweist, die nahe der Halsöffnung (20) angeordnet ist, wobei der ausgefüllte Raum (136) das Ankopplungssystem (30) ist.

7. Kleidungsstück nach Anspruch 6, wobei die Leitung (50) zumindest ein Schlauch (32) ist, der durch den ausgefüllten Raum (136) verläuft.

8. Kleidungsstück (10) nach Anspruch 6, wobei die Leitung (50) ein unteres erstes Anschlussstück (62), eine Vielzahl von in der Mitte befindlichen Schläuchen (60) und ein oberes zweites Anschlussstück (62) umfasst, wobei das obere zweite Anschlussstück (62) für die Ankopplung an eine derartige Gesichtsmaskenleitung (3) ausgelegt ist und wobei die Vielzahl von in der Mitte befindlichen Schläuchen (60) durch den ausgefüllten Raum (136) verläuft.

9. Kleidungsstück nach Anspruch 6, wobei die Innenschicht (162) und die Außenschicht (164) ein gasundurchlässiges Material enthalten und der ausgefüllte Raum einen Balg (150) bildet, der die Leitung (50) abgrenzt, wobei die Leitung Folgendes umfasst:
eine erste untere Öffnung (166) mit einem Leitungszwischenstück (168), das so strukturiert ist, dass es an eine derartige Zuführleitung (2) gekoppelt wird, und
eine zweite obere Öffnung (170) mit einem Leitungszwischenstück (168), das so strukturiert ist, dass es an eine derartige Gesichtsmaskenleitung (3) gekoppelt wird.

10. Kleidungsstück nach Anspruch 9, wobei der Balg (150) mit einer Matrix aus starrem Material gefüllt ist.

11. Kleidungsstück nach Anspruch 6, wobei die Innenschicht (162) und die Außenschicht (164) ein gasundurchlässiges Material enthalten und der ausgefüllte Raum eine Vielzahl von Bälgen (180) bildet, die die Leitung (50) abgrenzen.

12. Kleidungsstück nach Anspruch 1, wobei die Hülle (12) ferner Leggings (24) umfasst und die Leitung (50) ferner durch zumindest einen Teil der Leggings (24) verläuft.

## Revendications

1. Vêtement (10) comprenant :
(a) une couverture (12) structurée pour être disposée sur au moins une partie du haut du corps, dans lequel la couverture comprend un côté intérieur (16), un côté extérieur (18), une ouverture de col (20), et une partie de taille (22) ; et
(b) un système de gestion de tubes (14) comprenant :
(1) une conduite (50) s'étendant de la partie de taille à l'ouverture de col (20), la conduite (50) comprenant un tube flexible (52) couplé au côté intérieur ou au côté extérieur de la couverture, la conduite (50) étant adaptée pour être couplée de façon détectable à une conduite d'alimentation (2) et une conduite de masque facial (3) ; et
(2) un système de couplage (30) adapté pour coupler la conduite à la couverture, **caractérisé en ce que** le système de couplage (30) comprend un premier couplage en boucle (70) couplé au tube flexible et à la couverture, le premier couplage de boucle comprenant une boucle allongée (80) s'étendant entre l'ouverture de col (20) et la partie de taille (22).

2. Vêtement selon la revendication 1, dans lequel la conduite (50) comprend un premier collecteur inférieur (62), une pluralité de tubes flexibles (60) et un deuxième collecteur supérieur (62), le deuxième collecteur supérieur (62) étant couplé à la conduite de masque facial (3).

3. Vêtement selon la revendication 1, dans lequel la conduite (50) comprend un tube flexible (52) ayant une première extrémité inférieure, une partie médiane et une deuxième extrémité supérieure et dans lequel le système de couplage (30) comprend un premier matériau magnétique (110) couplé fonctionnellement à une partie de la couverture (12) et un deuxième matériau magnétique (112) couplé fonctionnellement à une partie de la conduite (50) de telle sorte que le premier matériau magnétique (110) et le deuxième matériau magnétique (112) interagissent pour coupler la conduite (50) à la couverture (12).

4. Vêtement selon la revendication 3, dans lequel la conduite (50) comprend au moins un manchon (122) constitué du deuxième matériau magnétique (112), dans lequel l'au moins un manchon (122) est fixé au tube flexible (52).

5. Vêtement selon la revendication 1, dans lequel le système de couplage (30) comprend une feuille allongée d'un premier matériau magnétique (110) adaptée pour être couplée à la couverture (12), dans lequel la conduite comprend un premier collecteur inférieur (62), une pluralité de tube flexibles médians (60) et un deuxième collecteur supérieur (62), le deuxième collecteur supérieur (62) étant structuré pour être couplé à cette conduite de masque facial (3) et dans lequel le système de couplage (30) comprend en outre une feuille d'un deuxième matériau magnétique (112) structurée de façon à être couplée à la feuille d'un premier matériau magnétique (110), les tubes médians (60) étant disposés entre elles.

6. Vêtement selon la revendication 1, dans lequel la couverture (12) a au moins une partie (160) dotée d'une couche intérieure (162) et d'une couche extérieure (164), les couches intérieure et extérieure formant une chambre (136) entre elles, la chambre (136) ayant une première ouverture inférieure (166) disposée à proximité de la partie de taille (22) et une deuxième ouverture supérieure (170) disposée à proximité de l'ouverture de col (20) et dans lequel la chambre (136) est le système de couplage (30).

7. Vêtement selon la revendication 6, dans lequel la conduite (50) est au moins un tube flexible (32) s'étendant dans la chambre (136).

8. Vêtement (10) selon la revendication 6, dans lequel la conduite (50) comprend un premier collecteur inférieur (62), une pluralité de tubes flexibles médians (60) et un deuxième collecteur supérieur (62), dans lequel le deuxième collecteur supérieur (62) est adapté pour être couplé à cette conduite de masque faciale (3) et dans lequel la pluralité de tubes flexibles médians (60) s'étend dans la chambre (136).

9. Vêtement selon la revendication 6, dans lequel la couche intérieure (162) et la couche extérieure (164) comprennent un matériau imperméable aux gaz et la chambre forme une poche (150) définissant la conduite (50) et la conduite comprenant :
une première ouverture inférieure (166) ayant une interface de conduite (168) structurée de façon à être couplée à cette conduite d'approvisionnement (2), et
une deuxième ouverture supérieure (170) ayant une interface de conduite (168) structurée pour être couplée à cette conduite de masque facial (3).

10. Vêtement selon la revendication 9, dans lequel la poche (150) est remplie avec une matrice d'un matériau rigide.

11. Vêtement selon la revendication 6, dans lequel la couche intérieure (162) et la couche extérieure (164) comprennent un matériau imperméable aux gaz et la chambre (136) forme une pluralité de poches (180) définissant la conduite (50).

12. Vêtement selon la revendication 1, dans lequel la couverture (12) comprend en outre des jambières (24) et la conduite s'étend en outre au travers d'au moins une partie des jambières (24).
